# EUROPÄISCHE PATENTANMELDUNG

(11) **EP 1 787 580 A1**
(43) Veröffentlichungstag der Anmeldung: **23.05.2007**
(21) Anmeldenummer: 06291714.1
(22) Anmeldetag: 06.11.2006
(51) Int. Cl.: A61B 5/022

(54) **Blutdruckmeßgerät mit einstellbaren Referenzwerten**

(30) Priorität: 07.11.2005 DE 202005017370 U
(71) Anmelder: Health & Life Co., Ltd., Chung Ho City, Taipei Hsien, R.O.C. (TW); Chinasia Products Ltd., Wan Chai (HK)
(72) Erfinder: Yang, Paul, Chung Ho City Taipei (TW)
(74) Vertreter: Blot, Philippe Robert Emile

(57) **Zusammenfassung**

Die Erfindung betrifft ein Blutdruckmeßgerät mit einstellbaren Referenzwerten, bestehend aus einem Manometer (40), einem Analog-Digital-Umsetzer (30), der mit dem Manometer (40) verbunden ist, einem Mikroprozessor (20), der mit dem Analog-Digital-Umsetzer (30) sowie einem Einstellelement (50), einem Display (60), einem Speichermedium (70), einem Warnelement (80), und einem Netzteil (10) verbunden ist. Beim Einsatz wird das Meßsignal des Manometers (40) von dem Analog-Digital-Umsetzer (30) in ein Digitalsignal umgesetzt und an den Mikroprozessor (20) gesendet, der die Meßwerte mit den im Speichermedium (70) gespeicherten Referenzwerten vergleicht und die Meßwerte auf dem Display (60) anzeigt, wobei der Benutzer die Einstellung der Referenzwerte durch das Einstellelement (50) verändern kann, und das Warnelement (80) eine optische oder akustische Warnung für das Meßergebnis erzeugen kann.

## Beschreibung

### Technisches Gebiet

Die Erfindung betrifft ein Blutdruckmeßgerät mit einstellbaren Referenzwerten, das eine Neueinstellung der Referenzwerte nach dem neuen Standard von WHO, JNC oder der entsprechenden Gesundheitsorganisation oder nach Instruktion des Arztes gestattet und eine optische oder akustische Warnung für das Meßergebnis erzeugen kann.

### Stand der Technik

Tragbare medizinische Meßgeräte, wie Blutzuckermeßgerät, elektronisches Ohr-Thermomenter, elektronisches Blutdruckmeßgerät, können von dem Benutzer zuhause selbst betätigt werden. Dadurch kann der Patient selbst seinen Gesundheitszustand überwachen.

Üblicherweise werden die Referenz- und Grenzwerten der medizinischen Meßgeräte vor der Lieferung von dem Hersteller nach dem Standard von WHO, JNC oder der entsprechenden Gesundheitsorganisation festgelegt. Mit der medizinischen Entwicklung modifizieren WHO, JNC und andere Gesundheitsorganisationen jedoch die Referenz- und Grenzwerten für den Blutdruck. Z.B. die siebte Kommission von JNC (JNC 7) hat die Refernz- und Grenzwerte der sechsten Kommission von JNC (JNC6) von systolischem/diastolischem Druck < 140/90mmHg auf systolischen/diastolischen Druck < 120/80mmHg modifiziert. Da die Referenz- und Grenzwerte der medizinischen Meßgeräte vor der Lieferung bereits von dem Hersteller festgelegt und unveränderbar sind, kann eine fehlerhafte Beurteilung des Gesundheitszustandes gemacht werden.

Wenn der Benutzer ein hypertonischer Patient ist, sind die Mittelwerte seines Blutdruckes höher als eine gesunde Person. Dabei sollen die Grenzwerte für ihn höher sein. Wenn der Benutzer ein hypotonischer Patient ist, sind die Mittelwerte seines Blutdruckes niedriger als eine gesunde Person. Dabei sollen die Grenzwerte für ihn niedriger sein. Dadurch kann eine unnötige Panik vermieden werden.

Das Blutdruckmeßgerät kann üblicherweise entsprechend den Blutdruckzustand eine geeignete Warnung geben: z.B. wenn der Benutzer ein hypertonischer Patient ist, wird für einen systolischen/diastolischen Druck< 120/80mmHg ein grünes Licht erzeugt, das einen normalen Zustand darstellt, wird für einen systolischen/diastolischen Druck = 120~139/80~89mmHg ein gelbes Licht erzeugt, das einen skeptischen Zustand darstellt, und wird für einen systolischen/diastolischen Druck> 14 0/90mmHg ein rotes Licht erzeugt, das einen ersthaftes Zustand darstellt. Wenn der Benutzer ein hypotonischer Patient ist, kann er keine Warnung bekommen, da das Blutdruckmeßgerät beim Unterschreiten eines bestimmten Grenzwertes keine Warnung gibt. Zudem läßt sich die Farbe des Warnlichtes schwer unterscheiden, wenn der Benutzer alt oder krank ist.

### Aufgabe der Erfindung

Der Erfindung liegt die Aufgabe zugrunde, ein Blutdruckmeßgerät mit einstellbaren Referenzwerten zu schaffen, das eine Neueinstellung der Referenz- und Grenzwerte nach dem neuen Standard von WHO, JNC oder der entsprechenden Gesundheitsorganisation oder nach Instruktion des Arztes gestattet.

Der Erfindung liegt eine weitere Aufgabe zugrunde, ein Blutdruckmeßgerät mit einstellbaren Referenzwerten zu schaffen, das ein Manometer aufweist, das mit einemAnalog-Digitl-Umsetzer verbunden ist, der mit einem Mikroprozessor verbunden ist, der mit einem Einstellelement, einem Display, einem Speichermedium, einem Warnelement, und einem Netzteil verbunden ist. Beim Einsatz wird das Meßsignal des Manometers von dem Analog-Digital-Umsetzer in ein Digitalsignal umgesetzt und an den Mikroprozessor gesendet, der die Meßwerte mit den im Speichermedium gespeicherten Referenzwerten vergleicht und die Meßwerte auf dem Display anzeigt, wobei der Benutzer die Einstellung der Referenzwerte durch das Einstellelement verändern kann, und das Warnelement eine optische oder akustische Warnung für das Meßergebnis erzeugen kann.

Der Erfindung liegt eine nochmals weitere Aufgabe zugrunde, ein Blutdruckmeßgerät mit einstellbaren Referenzwerten zu schaffen, wobei, wenn die neueingestellten Grenzwerte höher sind, der Benutzer als ein hypertonischer Patient definiert wird, wodurch eine entsprechende Warnung erzeugt werden kann, und wenn die neueingestellten Grenzwerte niedriger sind, der Benutzer als ein hypotonischer Patient definiert wird, wodurch eine entsprechende Warnung erzeugt werden kann.

Der Erfindung liegt eine nochmals weitere Aufgabe zugrunde, ein Blutdruckmeßgerät mit einstellbaren Referenzweren zu schaffen, wobei das Display eine LCD-Anzeige ist und zum Anzeigen der Meßergebnisse dient.

Der Erfindung liegt eine nochmals weitere Aufgabe zugrunde, ein Blutdruckmeßgerät mit einstellbaren Referenzwerten zu schaffen, wobei das Warnelement ein Hintergrundlichtmodul ist, der entsprechend den Meßergebnissen eine geeignete Warnung geben kann.

Weitere Einzelheiten, Merkmale und Vorteile der Erfindung ergeben sich aus der nachfolgenden detaillierten Beschreibung eines bevorzugten Ausführungsbeispiels in Verbindung mit den anliegenden Zeichnungen.

### Kurze Beschreibung der Zeichnungen

- Figur 1: eine schematische Darstellung der Erfindung,
- Figur 2: eine Blockschaltung der Erfindung,
- Figur 3: ein Flußbild der Erfindung.

### Wege zur Ausführung der Erfindung

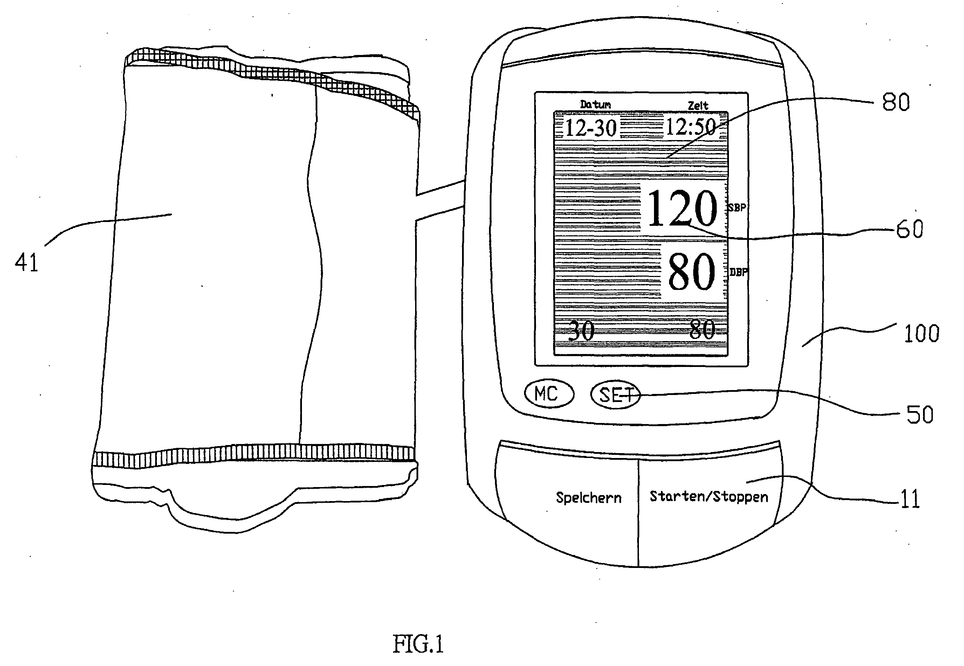

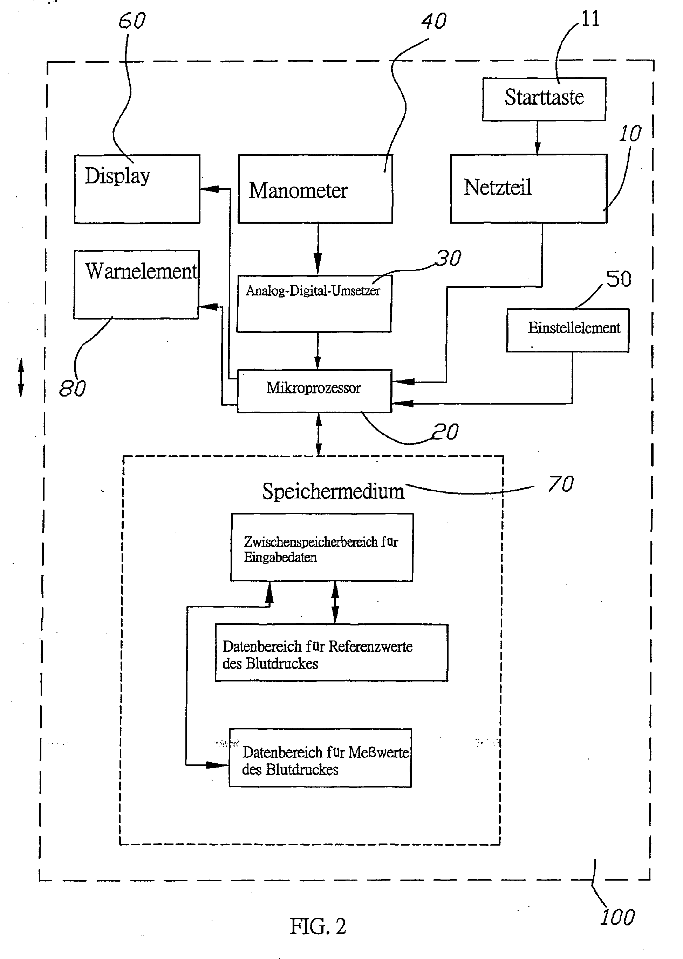

Wie aus den Figuren 1 und 2 ersichtlich ist, besteht die Erfindung aus einem Maschinenkörper 100, der eine beliebige Form haben kann und ein Netzteil 10 aufweist, das mit einer Starttaste 11 verbunden ist, durch den die Blutdruckmessung angesteuert werden kann.

Im Maschinenkörper 100 ist ein Mikroprozessor 20 vorgesehen, der mit dem Netzteil 10 und einem Anlag-Digital-Umsetzer 30 verbunden ist. Der Analog-Digital-Umsetzer 30 ist mit einem Manometer 40 verbunden, das wiederum mit einer Luftmanschette 41 verbunden ist, die um das Handgelenk oder den Arm des Benutzers gelegt und aufgepumpt werden kann, damit die Armschlagader abgedrosselt wird. Der Analog-Digital-Umsetzer 30 kann das Drucksignal aus dem Manometer 40 in ein Digitalsingal umsetzen.

Der Mikroprozessor 20 ist weiterhin mit einem Einstellelement 50, einem Display 60, einem Speichermedium 70 und einem Warnelement 80 verbunden. Das Display 60 ist eine LCD-Anzeige, auf der die Meßergebnisse angezeigt werden können. Das Einstellelement 50 ist eine Drucktaste, durch die die Einstellung der Referenzwerte des Blutdruckes verändert werden kann. Das Speichermedium 70 ist ein Speicher und kann die eingestellten Referenzwerte und die Meßergebnisse speichern. Das Warnelement 80 ist durch einen Hintergrundlichtmodul gebildet, der unterschiedliches Farblicht erzeugen kann. Der Benutzer kann die Referenz- und Grenzwerte nach dem neuen Stand der Gesundheitsorganisation neu einstellen. Wenn der Benutzer einen höheren Grenzwert einstellt, wird der Benutzer als ein hypertonischer Patient definiert, wodurch das Warnelement 80 dementsprechend eine Warnung erzeugt: z.B. ein grünes Licht aus dem Hintergrundlichtmodul für einen normalen Zustand des Blutdruckes (systolischer/diastolischer Druck <120/80mmHg), ein gelbes Licht aus dem Hintergrundlichtmodul für einen skeptischen Zustand des Blutdruckes (systolischer/diastolischer Druck = 120~139/80~89mmHg); und ein rotes Licht aus dem Hintergrundlichtmodul für einen erstenhaften Zustand des Blutdruckes (systolischer/diastolischer Druck > 140/90mmHg). Wenn der Benutzer einen niedrigen Grenzwert einstellt, wird der Benutzer als ein hypotonischer Patient definiert, wodurch das Warnelement 80 dementsprechend eine Warnung erzeugt: z.B. ein grünes Licht aus dem Hintergrundlichtmodul für einen norzmalen Zustand des Blutdruckes (systolischer/diastolischer Druck < 120/80mmHg), ein gelbes Licht aus dem Hintergrundlichtmodul für einen skeptischen Zustand des Blutdruckes (systolischer/diastolischer Druck = 110~100/70~75mmHg); und ein rotes Licht aus dem Hintergrundlichtmodul für einen erstenhaften Zustand des Blutdruckes (systolischer/diastolischer Druck < 100/70mmHg).

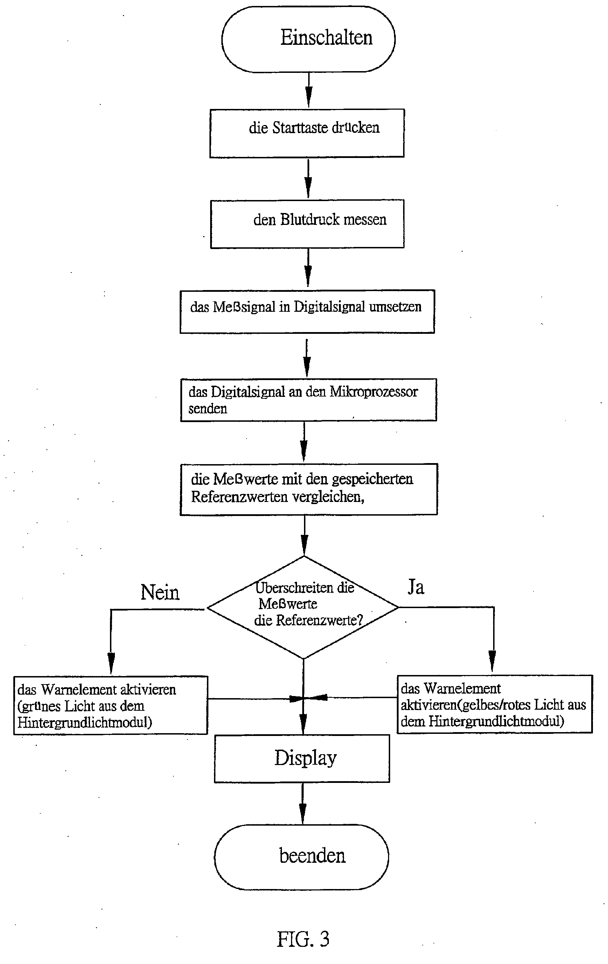

Beim Einsatz, wie es in den Figuren 1, 2 und 3 ersichtlich ist, wird zunächst das Blutdruckmeßgerät eingeschaltet, wodurch das Netzteil 10 Strom an die mit dem Netzteil 10 verbundenen Baulelemente liefert. Anschließend wird die Luftmanschette 41 um den Arm oder das Handgelenk des Patienten gelegt. Dann wird die Starttaste 11 betätigt, wodurch eine Pumpe (nicht dargestellt) die Luftmaschette 41 aufpumpt, bis die Luftmanschette 41 die Armschlagader abdrosselt. Danach wird die Pumpe abgeschaltet und ein Entlastungsventil geöffnet, wodurch die Luft langsam ausgelassen wird. Dabei kann das Manometer 40 den systolischen und diastolischer Blutdruck ermitteln. Das Meßsignal des Manometers 40 wird über den Analog-Digital-Umsetzer 30 an den Mikroprozessor 20 gesendet, der die Meßwerte auf dem Display 60 anzeigt und mit den im Speichermedium 70 gespeicherten Referenzwerten vergleicht. Wenn die Meßwerte die Referenzwerte unterschreiten, erzeugt das Warnelement 80 ein grünes Licht. Wenn die Meßwerte die Referenzwerte überschreiten, erzeugt das Warnelement ein Warnlicht (gelbes oder rotes Licht). Gleichzeitig werden die Meßdaten in dem Speichermedium 70 gespeichert.

Der Benutzer kann nach dem neuen Standard von WHO, JNC oder der entsprechenden Gesundheitsorganisation oder nach Instruktion des Arztes die Referenzwerte durch das Einstellelement 50 neueinstellen. Die neue Einstellung wird in dem Speichermedium 87 gespeichert. Beim Messen werden die Meßwerte mit den neuen Referenzwerten verglichen, auf dem Display 60 angezeigt und in dem Speichermedium 70 gespeichert, wobei das Warnelement 80 eine entsprechende Warnung geben kann.

Aufgrund der obengenannten Tatsachen entspricht die Erfindung in ihrer Verfügbarkeit, Fortschrittlichkeit und Neuheit vollauf den Anforderungen für ein Gebrauchsmuster.

### Bezugszeichenliste

- 100: Hauptkörper
- 10: Netzteil
- 11: Starttaste
- 20: Mikroprozessor
- 30: Analog-Digital-Umsetzer
- 40: Manometer
- 41: Luftmanschette
- 50: Einstellelement
- 60: Display
- 70: Speichermedium
- 80: Warnelement

## Patentansprüche

1. Blutdruckmeßgerät mit einstellbaren Referenzwerten, bestehend aus
einem Manometer (40), das den Blutdruck messen kann,
einem Analog-Digitl-Umsetzer (30), der mit dem Manometer (40) verbunden ist und das Meßsignal aus dem Manometer (40) in ein Digitalsignal umsetzen kann,
einem Mikroprozessor (20), der mit dem Analog-Digitl-Umsetzer (30) verbunden ist,
einem Display (60), das mit dem Mikroprozesser (20) verbunden ist und die Meßergebnisse anzeigen kann,
einem Einstellelement (50), das mit dem Mikroprozesser (20) verbunden ist und die Einstellung der Referenzwerte des Blutdruckes verändern kann,
einem Speichermedium (70), das mit dem Mikroprozesser (20) verbunden ist und die Referenzwerte und die Meßwerte speichern kann,
einem Warnelement (80), das mit dem Mikroprozesser (20) verbunden ist, durch einen Hintergrundlichtmodul gebildet ist und eine Warnung erzeugen kann, und
einem Netzteil (10), der das Manometer (40), den Mikroprozessor (20), das Display (60) und das Einstellelement (50) mit den erforderlichen Strom versorgt;
der Benutzer kann nach dem neuen Standard von WHO, JNC oder der entsprechenden Gesundheitsorganisation oder nach Instruktion des Arztes die Referenzwerte durch das Einstellelement (50) neueinstellen, und gleichzeitig können die neu eingestellten Grenzwerte mit den ursprünglichen Grenzwerte verglichen, um zu unterscheiden, ob der Benutzer ein hypertonischer oder hypotonischer Patient ist, wonach diese Daten in dem Speichermedium gespeichert werden; beim Messen werden die Meßwerte mit den neuen Referenzwerten verglichen und auf dem Display angezeigt, wobei eine entsprechende Warnung erzeugt werden kann.

2. Blutdruckmeßgerät nach Anspruch 1, **dadurch gekennzeichnet, dass** das Einstellelement (50) eine Drucktaste ist.

3. Blutdruckmeßgerät nach Anspruch 1, **dadurch gekennzeichnet, dass** das Display (60) eine LCD-Anzeige ist.

4. Blutdruckmeßgerät nach Anspruch 1, **dadurch gekennzeichnet, dass** das Speichermedium (70) ein externer Speicher ist.

5. Blutdruckmeßgerät nach Anspruch 1, **dadurch gekennzeichnet, dass** das Warnelement (80) neben dem Licht des Hintergrundlichtmoduls noch eine akustische Warnung geben kann.
